# EUROPEAN PATENT SPECIFICATION

(11) **EP 3 302 591 B1**
(45) Date of publication and mention of the grant of the patent: **30.12.2020**
(21) Application number: 15727929.0
(22) Date of filing: 29.05.2015
(51) Int. Cl.: A61L 27/26, A61L 27/52, C08B 37/02, C08B 37/08

(54) **MIXED HYDROGELS OF HYALURONIC ACID AND DEXTRAN**
GEMISCHTE HYDROGELE AUS HYALURONSÄURE UND DEXTRAN
HYDROGELS MÉLANGÉS D'ACIDE HYALURONIQUE ET DE DEXTRANE

(43) Date of publication of application: 11.04.2018
(73) Proprietor: Galderma S.A., 6330 Cham (CH)
(72) Inventor: KARLSSON, Anders, 743 40 Storvreta (SE); MOJARRADI, Hotan, 754 31 Uppsala (SE); SAWÉN, Elin, 192 72 Sollentuna (SE)
(74) Representative: AWA Sweden AB
(86) International application number: PCT/EP2015/062011
(87) International publication number: WO 2016/192760

(56) References cited:
- EP-A1- 3 040 348
- WO-A1-97/04012
- WO-A1-2014/174213
- WO-A1-2015/181365
- WO-A1-2015/181366
- WO-A1-2015/181369

## Description

### Technical field of the invention

The present invention relates to the field of hydrogels containing cross-linked polysaccharides and the use of such hydrogels in medical and/or cosmetic applications.

### Background of the invention

One of the most widely used biocompatible polymers for medical use is hyaluronic acid (HA). It is a naturally occurring polysaccharide belonging to the group of glycosaminoglycans (GAGs). Hyaluronic acid and the other GAGs are negatively charged heteropolysaccharide chains which have a capacity to absorb large amounts of water. Hyaluronic acid and products derived from hyaluronic acid are widely used in the biomedical and cosmetic fields, for instance during viscosurgery and as a dermal filler.

Water-absorbing gels, or hydrogels, are widely used in the biomedical field. They are generally prepared by chemical cross-linking of polymers to infinite networks. While native hyaluronic acid and certain cross-linked hyaluronic acid products absorb water until they are completely dissolved, cross-linked hyaluronic acid gels typically absorb a certain amount of water until they are saturated, i.e. they have a finite liquid retention capacity, or swelling degree.

Since hyaluronic acid is present with identical chemical structure except for its molecular mass in most living organisms, it gives a minimum of reactions and allows for advanced medical uses. Cross-linking and/or other modifications of the hyaluronic acid molecule is necessary to improve its duration *in vivo.* Furthermore, such modifications affect the liquid retention capacity of the hyaluronic acid molecule. As a consequence thereof, hyaluronic acid has been the subject of many modification attempts.

WO 97/04012 discloses a process of preparing a cross-linked polysaccharide product comprising hyaluronic acid and dextranomer (i.e. a crosslinked dextran).

### Summary of the invention

It is an object of the present invention to provide a cross-linked polysaccharide product suitable for use as a dermal filler.

It is a further object of the present invention to provide a cross-linked polysaccharide product suitable having improved durability in use as a dermal filler.

For these and other objects that will be evident from this disclosure, the present invention provides according to a first aspect a process, as defined in claim 1, of preparing a cross-linked polysaccharide product comprising hyaluronic acid and dextran.

The cross-linked polysaccharide products according to the invention can be used, e.g., as injectable compositions for cosmetic or medical surgery, like dermal filling and body contouring. The cross-linked polysaccharide products according to the invention, combining hyaluronic acid with dextran, have better stability to heat degradation as well as to radical and enzymatic degradation by, for instance by chondroitinase and hyaluronidase, as compared hyaluronic acid products without dextran. A possible explanation is that the hyaluronic acid backbone is protected by the dextran. Susceptibility to enzymatic degradation is likely decreased due to steric hindrance. This leads to an improved of durability *in vivo* of the cross-linked polysaccharide products according to the invention as compared hyaluronic acid products without dextran.

The dextran is attached to the hyaluronic acid by ether bonds. The use of ether bonds in the dextran-hyaluronic acid linkage (graft) has been found to be advantageous compared to e.g. ester bonds, since the ether bond is more stable to degradation *in vivo.*

Step (b) comprises cross-linking the dextran to the hyaluronic acid by ether bonds using a bi- or polyfunctional cross-linking agent.

The hyaluronic acid provided in step (a) is a cross-linked hyaluronic acid gel, and the dextran provided in step (a) is a non cross-linked dextran.

According to some embodiments, the dextran provided in step (a) is a dextran pre-activated with a bi- or polyfunctional cross-linking agent such that the dextran comprises at least one bi- or polyfunctional cross-linking agent bound thereto having at least one functional group available for grafting the dextran to the hyaluronic acid.

According to a second aspect illustrated herein, there is provided a cross-linked polysaccharide product as defined in claim 12.

Since the nature of the product obtainable by the processes according to the invention is complex, the product may also be defined as being the result of these processes. According to another aspect illustrated herein, there is provided a cross-linked polysaccharide product comprising hyaluronic acid and dextran, obtainable by the process decribed herein with reference to the first aspect.

The cross-linked polysaccharide products of the present disclosure may for example be used in injectable formulations for treatment of soft tissue disorders, including but not limited to, corrective and aesthetic treatments.

The cross-linked polysaccharide products of the present disclosure may for example be used in injectable formulations for cosmetic surgery, e.g. dermal filling, body contouring and facial contouring, in medical surgery, e.g. dermal filling, body contouring, prevention of tissue adhesion, formation of channels, incontinence treatment, and orthopaedic applications, and for hydrating and/or vitalizing the skin.

The cross-linked polysaccharide product may also be provided in an injectable dermal aesthetic or pharmaceutical formulation.

The cross-linked polysaccharide product, or injectable formulation comprising a cross-linked polysaccharide product, as decribed herein may advantageously be used as a dermal filler.

According to aspects illustrated herein, there is provided a method of cosmetically treating skin, which comprises administering to the skin a cross-linked polysaccharide product as described herein.

Other aspects and preferred embodiments of the present invention will be evident from the following detailed disclosure of the invention and the appended claims.

### Detailed description of the invention

The present invention generally provides a cross-linked polysaccharide product, as defined in claims 12 and 13, comprising hyaluronic acid (also referred to herein as HA or hyaluronan) and a dextran bound to each other by a bi- or polyfunctional cross-linking agent and a process of preparing a cross-linked polysaccharide product comprising hyaluronic acid and dextran, as defined in claim 1.

The term cross-linking as used herein refers to a reaction involving sites or groups on existing macromolecules or an interaction between existing macromolecules that results in the formation of a small region in a macromolecule from which at least four chains emanate. A reaction of a reactive chain end of a linear macromolecule with an internal reactive site of another linear macromolecule results in the formation of a branch point or graft, but is not regarded as a cross-linking reaction.

The term grafting as used herein refers to a reaction in which one or more species of block are connected to the main chain of a macromolecule as side-chains having constitutional or configurational features that differ from those in the main chain.

Step (b) comprises cross-linking the dextran to the hyaluronic acid by ether bonds using a bi- or polyfunctional cross-linking agent.

High or low molecular weight non cross-linked hyaluronic acid and high or low molecular weight non cross-linked dextran can be cross-linked to form a mixed polymer hydrogel connected by ether bonds using a bi- or polyfunctional cross-linking agent, e.g. a diepoxide like butanediol diglycidyl ether (BDDE) or divinyl sulfone. The cross-linking reaction takes place between any of the free hydroxyl groups on dextran and hyaluronic acid. This reaction is shown in Reaction scheme 1. (not covered by the claims).

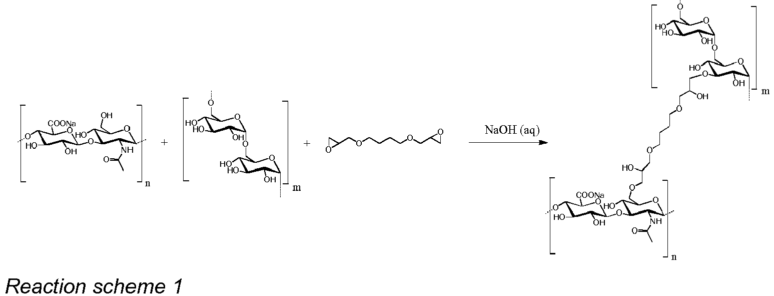

According to the embodiments claimed, the hyaluronic acid provided in step (a) is a cross-linked hyaluronic acid gel, and the dextran provided in step (a) is a non cross-linked dextran.

Non cross-linked dextran can be grafted to a cross-linked hyaluronic acid gel by ether bonds using a bi- or polyfunctional cross-linking agent, e.g. a diepoxide like butanediol diglycidyl ether (BDDE) or divinyl sulfone. The reaction takes place on any of the free hydroxyl groups on dextran and hyaluronic acid. This reaction is shown in Reaction scheme 2.

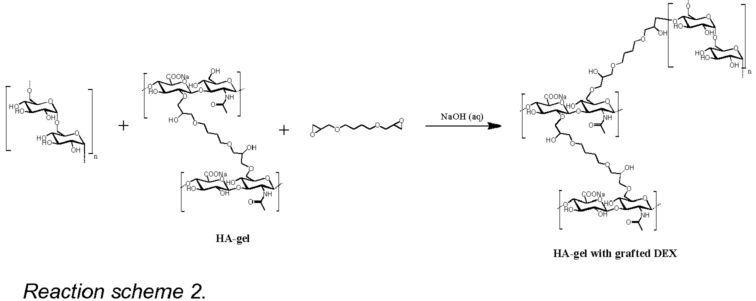

It is advantageous to graft the dextran molecules on already cross-linked hyaluronic acid, which may already be prepared in a desirable form having defined physico-chemical properties. This allows for a significant modification of cross-linked HA with dextran without inducing depolymerisation of the cross-linked HA.

According to some embodiments, the hyaluronic acid provided in step (a) is in the form of gel particles having an average swelled size (unless specified otherwise, all particle sizes given herein refer to weight average particle size) in the range of 0.01-5 mm, preferably 0.1-1 mm.

According to some embodiments, the dextran provided in step (a) is a dextran pre-activated with a bi- or polyfunctional cross-linking agent such that the dextran comprises at least one bi- or polyfunctional cross-linking agent bound thereto having at least one functional group available for grafting the dextran to the hyaluronic acid.

Dextran can be pre-activated by reaction of dextran and a diepoxide, where part of the diepoxide is still in its non-hydrolyzed epoxyform. Dextran substituted with a sidechain with an epoxy end-group can be grafted on to an HA-gel. Cross-links keeping the polymer network together will be present between the HA-chains. The dextran will be grafted on the cross-linked HA-chains by ether bonds. These reactions are shown in Reaction scheme 3. 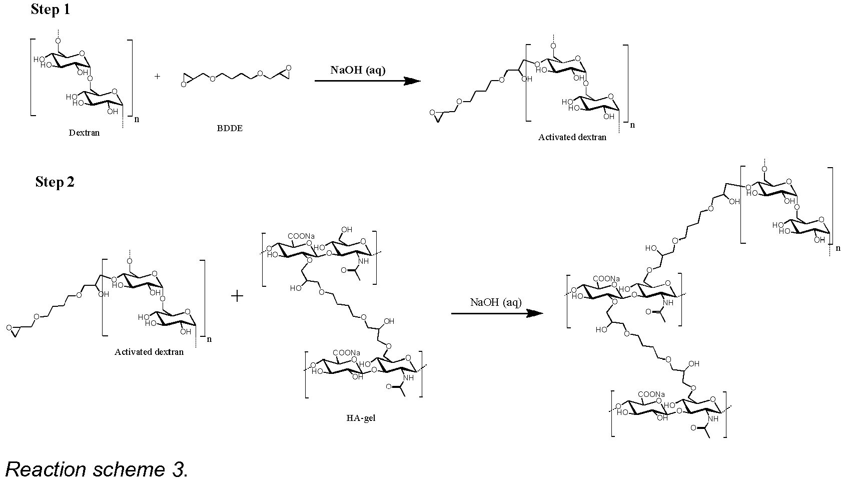 Pre-activated dextran can also be grafted to non cross-linked HA chains (embodiment not covered by the claims).

The formed HA-dextran copolymer is then subsequently cross-linked to form a mixed polymer hydrogel connected by ether bonds using a bi- or polyfunctional cross-linking agent, e.g. a diepoxide like butanediol diglycidyl ether (BDDE) or divinyl sulfone. The cross-linking reaction takes place between any of the free hydroxyl groups on dextran and hyaluronic acid.

As an alternative (embodiment not covered by the claims), the dextran can be grafted to an HA-gel by ester bonds by performing the reaction at a different pH. This reaction is shown in Reaction scheme 4.

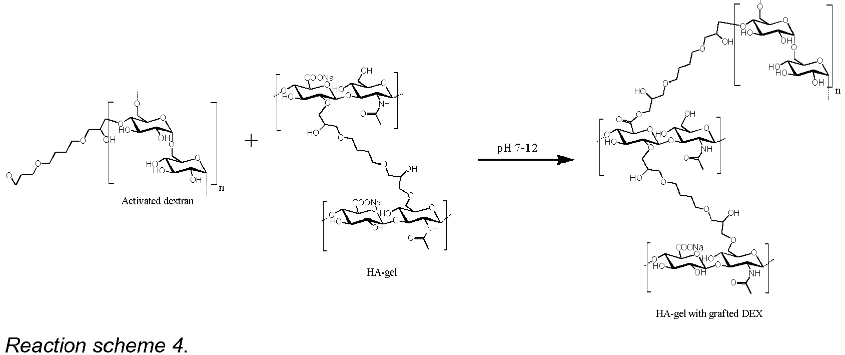

According to some embodiments, the dextran provided in step (a) has an average molecular weight of less than 10 kDa, preferably less than 5 kDa.

According to some embodiments, the hyaluronic acid provided in step (a) has an average molecular weight of less than 10 kDa, preferably less than 5 kDa.

According to some embodiments, the polysaccharide product is in the form of gel particles having an average size in the range of 0.01-5 mm, preferably 0.1-1 mm.

According to some embodiments, the bi- or polyfunctional cross-linking agent is divinyl sulfone or a bis- or polyepoxide.

According to some embodiments, the bi- or polyfunctional cross-linking agent is a bis- or polyepoxide.

According to some embodiments, the bi- or polyfunctional cross-linking agent is a diglycidyl ether.

According to some embodiments, the bi- or polyfunctional cross-linking agent is selected from the group consisting of 1,4-butanediol diglycidyl ether (BDDE), 1,2-bis(2,3-epoxypropoxy)ethylene (EGDGE) and ethylene glycol diglycidyl ether (EGDE), 1,2-ethanediol diglycidyl ether (EDDE) and diepoxyoctane.

According to some embodiments, the bi- or polyfunctional cross-linking agent is 1,4-butanediol diglycidyl ether (BDDE).

According to aspects illustrated herein, there is provided a cross-linked polysaccharide product comprising a hyaluronic acid and a dextran, wherein the hyaluronic acid is in the form of gel particles having an average size in the range of 0.01-5 mm, preferably 0.1-1 mm, and the dextran is grafted to a surface of gel particles by means of a bi- or polyfunctional cross-linking agent.

Since the nature of the product obtainable by the processes according to the invention is complex, the product may also be defined as being the result of these processes. According to aspects illustrated herein, there is provided a cross-linked polysaccharide product comprising hyaluronic acid and dextran, obtainable by the process decribed herein with reference to the first aspect.

The polysaccharide products were evaluated by their swelling, i.e. their ability to absorb water. Swelling is expressed as the amount of water in gram that one gram dry product can absorb. The swelling of the hyaluronic acid product is preferably in the range 0.5-10 mL/g, preferably in the range 2-5 mL/g.

The cross-linked polysaccharide product is preferably biocompatible. This implies that no, or only very mild, immune response occurs when the cross-linked polysaccharide product is introduced into the tissue of an individual. That is, no or only very mild undesirable local or systemic effects occur in the treated individual.

The cross-linked polysaccharide products of the present disclosure may for example be used in injectable formulations for treatment of soft tissue disorders, including but not limited to, corrective and aesthetic treatments.

The cross-linked polysaccharide products of the present disclosure may for example be used in injectable formulations for cosmetic surgery, e.g. dermal filling, body contouring and facial contouring, in medical surgery, e.g. dermal filling, body contouring, prevention of tissue adhesion, formation of channels, incontinence treatment, and orthopaedic applications, and for hydrating and/or vitalizing the skin.

According to aspects illustrated herein, there is provided a method of cosmetically treating skin, which comprises administering to the skin a cross-linked polysaccharide product as described herein.

The cross-linked polysaccharide product may also be provided in an injectable dermal aesthetic or pharmaceutical formulation.

The cross-linked polysaccharide products of the present disclosure may also be used in injectable formulations for the transport or administration and slow or controlled release of various pharmaceutical or cosmetic substances.

The injectable formulations may optionally include one or more other pharmaceutically acceptable components, including, but not limited to, buffers, preservatives, tonicity adjusters, salts, antioxidants, osmolality adjusting agents, emulsifying agents, wetting agents, sweetening or flavoring agents, and the like.

The injectable formulations may optionally include a pharmaceutically effective amount of an anesthetic agent. The anesthetic agent may be a local anesthetic agent, e.g. an aminoamide local anesthetic or aminoester local anesthetic. Examples of anesthetic agents include, but are not limited to, lidocaine, ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cyclomethycaine, dibucaine, dimethisoquin, dimethocaine, diperodon, dicyclomine, ecgonidine, ecgonine, ethyl chloride, etidocaine, β-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicyl alcohol, tetracaine, tolycaine, trimecaine, zolamine, combinations thereof, and salts thereof. Examples of aminoester local anesthetics include, but are not limited to procaine, chloroprocaine, cocaine, cyclomethycaine, dimethocaine (larocaine), propoxycaine, procaine (novocaine), proparacaine, tetracaine (amethocaine). Non-limiting examples of aminoamide local anesthetics include articaine, bupivacaine, cinchocaine (dibucaine), etidocaine, levobupivacaine, lidocaine (lignocaine), mepivacaine, piperocaine, prilocaine, ropivacaine, trimecaine, or a combination thereof.

The cross-linked polysaccharide product, or injectable pharmaceutical formulation comprising a cross-linked polysaccharide product, as decribed herein may be used for improving the appearance of skin, filling wrinkles or contouring the face or body of a subject.

The cross-linked polysaccharide product, or injectable pharmaceutical formulation comprising a cross-linked polysaccharide product, as decribed herein may advantageously be used as a dermal filler.

The cross-linked polysaccharide product, or injectable pharmaceutical formulation comprising a cross-linked polysaccharide product, as decribed herein may be used in a method of cosmetically treating skin, which comprises administering to the skin a cross-linked polysaccharide product as described herein.

The cross-linked polysaccharide product, or injectable pharmaceutical formulation comprising a cross-linked polysaccharide product, as decribed herein may also be used in the treatment of a joint disorder by intraarticular injection.

The above described uses of the cross-linked polysaccharide product, or injectable pharmaceutical formulation comprising a cross-linked polysaccharide product, may be medical procedures or purely cosmetic non-medical procedures.

Unless otherwise provided, the term hyaluronic acid (also referred to herein as HA or hyaluronan) encompasses all variants and combinations of variants of hyaluronic acid, hyaluronate or hyaluronan, of various chain lengths and charge states, as well as with various chemical modifications. That is, the term also encompasses the various hyaluronate salts of hyaluronic acid with various counter ions, such as sodium hyaluronate. Various modifications of the hyaluronic acid are also encompassed by the term, such as oxidation, e.g. oxidation of -CH₂OH groups to -CHO and/or - COOH; periodate oxidation of vicinal hydroxyl groups, optionally followed by reduction, e.g. reduction of -CHO to -CH₂OH or coupling with amines to form imines followed by reduction to secondary amines; sulphation; deamidation, optionally followed by deamination or amide formation with new acids; esterification; and deacetylation. Other examples of modifications are isourea, hydrazide, bromocyan, monoepoxide and monosulfone couplings.

The hyaluronic acid can be obtained from various sources of animal and non-animal origin. Sources of non-animal origin include yeast and preferably bacteria. The molecular weight of a single hyaluronic acid molecule is typically in the range of 0.1-10 MDa, but other molecular weights are possible.

In certain embodiments, the concentration of the cross-linked hyaluronic acid is in the range of 1 to 100 mg/ml. In some embodiments the concentration of the cross-linked hyaluronic acid is in the range of 2 to 50 mg/ml. In specific embodiments the concentration of the cross-linked hyaluronic acid is in the range of 5 to 30 mg/ml or in the range of 10 to 30 mg/ml.

Cross-linked hyaluronic acid comprises cross-links between the hyaluronic acid chains, which creates a continuous network of hyaluronic acid molecules which is held together by the covalent cross-links, physical entangling of the hyaluronic acid chains and various interactions, such as electrostatic interactions, hydrogen bonding and van der Waals forces. Cross-linking of the hyaluronic acid may be achieved by modification with a cross-linking agent. The hyaluronic acid concentration and the extent of cross-linking affects the mechanical properties, e.g. the elastic modulus G', and stability properties of the gel. Cross-linked hyaluronic acid gels are often characterized in terms of "degree of modification". The degree of modification of hyaluronic acid gels generally range between 0.1 and 15 mole%. The degree of modification (mole%) describes the amount of cross-linking agent(s) that is bound to HA, i.e. molar amount of bound cross-linking agent(s) relative to the total molar amount of repeating HA disaccharide units. The degree of modification reflects to what degree the HA has been chemically modified by the cross-linking agent. Reaction conditions for cross-linking and suitable analytical techniques for determining the degree of modification are all well known to the person skilled in the art, who easily can adjust these and other relevant factors and thereby provide suitable conditions to obtain a degree of modification in the range of 0.1-2% and verify the resulting product characteristics with respect to the degree of modification. A BDDE (1,4-butandiol diglycidylether) cross-linked hyaluronic acid gel may for example be prepared according to the method described in Examples 1 and 2 of published international patent application WO 9704012.

In a preferred embodiment, the cross-linked hyaluronic acid is present in the form of a gel cross-linked by a cross-linking agent, wherein the concentration of said hyaluronic acid is in the range of 10 to 30 mg/ml, and the degree of modification with said cross-linking agent is in the range of 0.1 to 2 mole%.

Hyaluronic acid gels may also comprise a portion of hyaluronic acid which is not cross-linked, i.e not bound to the three-dimensional cross-linked hyaluronic acid network. However, it is preferred that at least 50 % by weight, preferably at least 60 % by weight, more preferably at least 70 % by weight, and most preferably at least 80 % by weight, of the hyaluronic acid in a gel composition form part of the cross-linked hyaluronic acid network.

The dextran may be of any average molecular weight (unless otherwise specified, all average molecular weights of dextran given herein refer to number average molecular weights, Mₙ), typically in the range of 0.2 to 3000 kDa In some embodiments it is preferred that the dextran has a lower molecular weight, such as less than 100 kDa, less than 50kDa, less than 25 kDa, less than 10 kDa or less than 5 kDa. The dextran has a molecular weight of more than 0.2 kDa, preferably mer than 0.5 kDa. In some embodiments, the dextran has a molecular weight in the range of 10-100 kDa or in the range of 10-50 kDa. In some preferred embodiments, the dextran has a molecular weight in the range of 0.5-10 kDa or in the range of 0.5-5 kDa. In one preferred embodiment, the dextran has an average molecular weight in the range of 0.5-3 kDa.

Dextrans are often chemically modified in order to improve their solubility in water and/or to optimize their performance in a specific application. The term dextran as used herein is also intended to encompass the functionally equivalent variants or derivatives thereof.

The cross-linked polysaccharide product according to the invention may be a gel, or a hydrogel. That is, it can be regarded as a water-insoluble, but substantially dilute cross-linked system of polysaccharide molecules when subjected to a liquid, typically an aqueous liquid.

The gel contains mostly liquid by weight and can e.g. contain 90-99.9% water, but it behaves like a solid due to a three-dimensional cross-linked polysaccharide network within the liquid. Due to its significant liquid content, the gel is structurally flexible and similar to natural tissue, which makes it very useful as a scaffold in tissue engineering and for tissue augmentation.

The cross-linked polysaccharide product may be present in the form of particles, strings, discs, etc. In a preferred embodiment, the cross-linked polysaccharide product is in the form of gel particles. The gel particles preferably have an average size in the range of 0.01-5 mm, preferably 0.1-1 mm, such as 0.2-0.5 mm or 0.5-0.8 mm.

The cross-linked polysaccharide product may be present in an aqueous solution, but it may also be present in dried or precipitated form, e.g. in ethanol. The cross-linked polysaccharide product is preferably injectable.

In the cross-linked polysaccharide product comprising dextran cross-linked to hyaluronic acid, the total polysaccharide concentration may be in the range 5-100 mg/mL, preferably in the range 15-40 mg/mL. The amount of dextran in the cross-linked polysaccharide product may be in the range of 5-95% by weight (based on the total dry weight of polysaccharide).

In the cross-linked polysaccharide product comprising dextran grafted to a cross-linked hyaluronic acid, the total polysaccharide concentration may be in the range 5-100 mg/mL, preferably in the range of 15-40 mg/mL. The weight amount of dextran grafted to the cross-linked hyaluronic acid is in the range of 0.1-50% by weight, preferably in the range of 0.5-25% by weight (based on the total dry weight of polysaccharide).

The hyaluronic acid chains are cross-linked to each other via a linking group which is derived from a bi- or polyfunctional cross-linking agent. The bi- or polyfunctional cross-linking agent of the connects the hyaluronic acid chains to each other. The bi- or polyfunctional cross-linking agent further acts as a spacer between the hyaluronic acid and/or dextran chains.

The bi- or polyfunctional cross-linking agent comprises two or more functional groups capable of reacting with functional groups of the hyaluronic acid, resulting in the formation of covalent bonds. The bi- or polyfunctional cross-linking agent may for example selected from the group consisting of divinyl sulfone, diepoxides and multiepoxides.

A preferred type of bi- or polyfunctional cross-linking agent is a bis- or polyepoxide, such as a diglycidyl ether. According to an embodiment, the bi- or polyfunctional cross-linking agent comprises two or more glycidyl ether functional groups. The glycidyl ether functional groups react with primary hydroxyl groups of the hyaluronic acid and/or dextran, resulting in the formation of ether bonds. It follows that when a diglycidyl ether cross-linking agent reacts with the primary hydroxyl groups of hyaluronan and/or dextran, two ether bonds are formed with an intermediate spacer remaining from the cross-linking agent.

Preferred bi- or polyfunctional cross-linking agent for cross-linking the hyaluronic acid chains include 1,4-butanediol diglycidyl ether (BDDE), 1,2-bis(2,3-epoxypropoxy)ethylene (EGDGE) and ethylene glycol diglycidyl ether (EGDE), 1,2-ethanediol diglycidyl ether (EDDE) and diepoxyoctane. A particularly preferred bi- or polyfunctional cross-linking agent is BDDE.

The cross-linked polysaccharide products of the present disclosure can be used, e.g., as injectable compositions for cosmetic or medical surgery, like dermal filling and body contouring. The cross-linked polysaccharide products according to the invention, combining hyaluronic acid with dextran, have been found to have a better stability to radical and enzymatic degradation as compared hyaluronic acid products without dextran. A possible explanation is that the hyaluronic acid backbone is protected by the dextran. This leads to an improved of durability *in vivo* of the cross-linked polysaccharide products according to the invention as compared hyaluronic acid products without dextran.

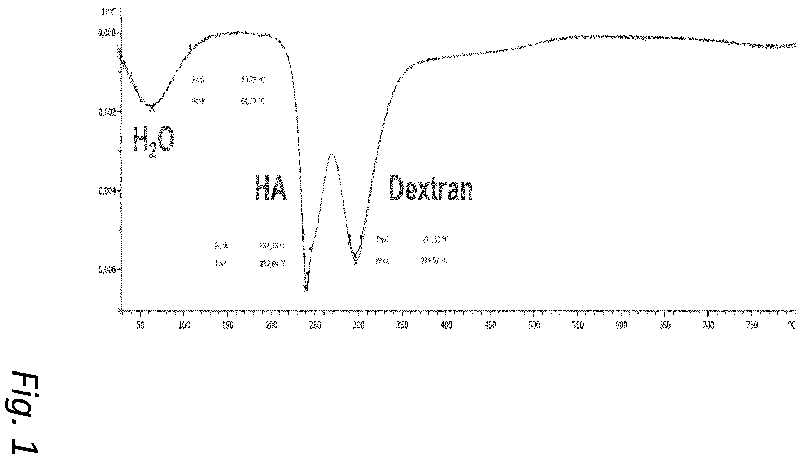

The dextran grafts are expected to protect the hydrogel backbone from heat degradation as dextran is more stable to degradation than hyaluronic acid. The thermal stability of dextran has been shown to be much higher than that of hyaluronic acid based on results from thermal gravimetric analysis of a mixed hydrogel of hyaluronic acid and dextran (Fig. 1).

In the disclosed cross-linked polysaccharide products, the dextran is preferably attached to the hyaluronic acid by ether bonds. The use of ether bonds in the dextran-hyaluronic acid linkage (graft) has been found to be advantageous compared to e.g. ester bonds, since the ether bond is more stable to degradation *in vivo.*

Without desiring to be limited thereto, the present invention will in the following be illustrated by way of examples.

### REFERENCE EXAMPLES

### Characterization of gels

The gels obtained in the following Examples were evaluated by the swelling, i.e. their ability to absorb water, and their viscoelastic properities. Swelling is expressed as the amount of water in mL that one gram dry polymer can absorb. The viscoelastic properties were measured by rheometry, and are expressed as the storage modulus (G') and the loss modulus (G").

The chemical composition of the HA-dextran gels was evaluated by proton NMR spectroscopy after degradation of the HA polysaccharide strands by hylauronidase or equivalent to obtain sharp lines in the spectrum enabling proper quantification.

The chemical link between HA and dextran was characterized by size exclusion chromatography coupled to mass spectrometry after degradation by both hylauronidase and dextranase or equivalent.

### Example 1a: HA (1 MDa) - dextran (500 kDa)

### Experiment

Dextran (500 kDa) was dissolved in 0.25 M NaOH in a 50 mL Falcon tube. HA (1 MDa) was added to the dextran solution and vigorously mixed. 0.1 mmol BDDE per gram polysaccharide was added to the dextran-HA mixture. The cross-linking and the treatment of the resulting material were done according to the general procedure described in Examples 1 and 2 of international patent application WO 97/04012 (Agerup et al.).

### Characterization

The gel content of the gel was between 70 and 80 % with a concentration of dextran of 9-11 mg/mL and a concentration of HA of 13-14 mg/ml. The total concentration of polysaccharide was 23-25 mg/mL. The degree of modification (MoD) was between 1.1 and 1.2 %.

### Example 1b - HA (1 MDa) - dextran (500 kDa)

### Experiment

Twelve gels using dextran 500 kDa and HA 1 MDa were made with varying concentrations of BDDE and NaOH, see the values in the table below. The cross-linking and the treatment of the resulting material were done according to the general procedure described in Examples 1 and 2 of international patent application WO 97/04012 (Ågerup *et al*.).

### Characterization

Swelling factor and rheometry (G' at 0.1 Hz) were analyzed and the results are presented in the Table 1.

**Table 1.**

| Experiment | mmol [BDDE] / g polysaccharide | [NaOH] (M) | Swelling factor (mL/g) | G' (Pa) |
|---|---|---|---|---|
| 1 | 0.03 | 0.8 | 2.1 | 0.5 |
| 2 | 0.04 | 1.3 | 2.9 | 0.5 |
| 3 | 0.05 | 1.8 | 3.0 | 0.4 |
| 4 | 0.03 | 1.3 | 3.5 | 0.2 |
| 5 | 0.03 | 1.8 | 4.4 | 0.1 |
| 6 | 0.04 | 1.8 | 3.6 | 0.2 |
| 7 | 0.05 | 1.3 | 2.7 | 0.6 |
| 8 | 0.07 | 1.8 | 2.8 | 0.5 |
| 9 | 0.06 | 2.8 | 3.1 | 0.2 |
| 10 | 0.07 | 2.8 | 2.8 | 0.3 |
| 11 | 0.08 | 2.8 | 2.4 | 0.4 |
| 12 | 0.087 | 2.75 | 2.2 | 428 |

### Example 2: HA (1 MDa) - dextran (1 kDa)

### Experiment

Dextran (1 kDa) was dissolved in 0.25 M NaOH. HA (1 MDa) was added to the solution. 0.1 mmol BDDE per gram polysaccharide was added to the dextran/HA mixture. The cross-linking and the treatment of the resulting material were done according to the general procedure described in Examples 1 and 2 of international patent application WO 97/04012 (Ågerup *et al*.).

### Characterization

The gel content for dextran of the gel is 15 % and 80 % for HA with a concentration of dextran of 9 mg/mL and a concentration of HA of 37 mg/ml. The total concentration of polysaccharide is 45 mg/mL. The degree of modification (MoD) was 4.2 %.

### Example 3: HA (66 kDa) - dextran 1 kDa

### Experiment

Five gels using dextran 1 kDa dextran and HA 70 kDa were made with varying concentrations of BDDE and 1.3 M NaOH, see the values in table 2 below. The cross-linking and the treatment of the resulting material were done according to the general procedure described in Examples 1 and 2 of international patent application WO 97/04012 (Ågerup *et al*.).

### Characterization

The gel content and concentration of dextran and HA are shown in table 2. The gel content describes how much of the respective polysaccharide that is incorporated in the gel network. A low value means that most of the polysaccharide is not in the gel network. The degree of modification (MoD) is between 9.3 and 15.5%.

**Table 2.**

| Experiment | mmol [BDDE] / g polysaccharide | [HA] (mg/mL) | [dextran] (mg/mL) | GelC HA (%) | GelC dextran (%) | MoD (%) |
|---|---|---|---|---|---|---|
| 1 | 0.06 | No gel obtained, analysis not continued | | | | |
| 2 | 0.11 | No gel obtained, analysis not continued | | | | |
| 3 | 0.17 | 9 | 7 | 52 | 5 | 9,3 |
| 4 | 0.22 | 11 | 7 | 74 | 16 | 11.7 |
| 5 | 0.28 | 10 | 7 | 84 | 18 | 15.5 |

### Example 4: Activation of dextran 1 kDa with BDDE followed by grafting to a HA-gel

### Experiment

40 g 0.25 M NaOH and 0.8 g BDDE was mixed in a glass bottle. 0.5 g of dextran 1 kDa was weighed in a plastic bottle. 2 g of the NaOH-BDDE solution was added to the dextran and then mixed thoroughly. The reaction was performed for 7 h at room temperature. Afterwards, 0.1 g of a precipitated HA-gel obtained according to the procedure described in Examples 1 and 2 of international patent application WO 97/04012 (Agerup et al.) was added to the reaction mixture followed by thorough mixing. The mixture was allowed to react for another day at room temperature. Afterwards the material was swelled in 45 g water and the pH was adjusted to 7 with acetic acid. The gel was thoroughly washed with 0.9% NaCl to remove excess of dextran and BDDE.

### Characterization

The amount of dextran grafted to HA, degree of modification (mole of dextran chains/mole of HA disaccharide repeating units), was equal to 0.4%.

## Claims

1. A process of preparing a cross-linked polysaccharide product comprising hyaluronic acid and dextran, the process comprising the steps of:
(a) providing a hyaluronic acid and a dextran;
(b) binding the dextran to the hyaluronic acid by ether or ester bonds using a bi- or polyfunctional cross-linking agent;
wherein the hyaluronic acid provided in step (a) is a cross-linked hyaluronic acid gel, and the dextran provided in step (a) is a non cross-linked dextran; and wherein step (b) comprises cross-linking the non cross-linked dextran to the hyaluronic acid by ether bonds using a bi- or polyfunctional cross-linking agent.

2. The process according to claim 1, wherein the hyaluronic acid provided in step (a) is in the form of gel particles having an average size in the range of 0.01-5 mm, preferably 0.1-1 mm.

3. The process according to any one of the preceding claims, wherein the dextran provided in step (a) is a dextran pre-activated with a bi- or polyfunctional cross-linking agent such that the dextran comprises at least one bi- or polyfunctional cross-linking agent bound thereto having at least one functional group available for grafting the dextran to the hyaluronic acid.

4. The process according to any one of of the preceding claims, wherein the dextran provided in step (a) has an average molecular weight of less than 10 kDa, preferably less than 5 kDa.

5. The process according to any one of the preceding claims, wherein the hyaluronic acid provided in step (a) is a hyaluronic acid pre-activated with a bi- or polyfunctional cross-linking agent such that the hyaluronic acid comprises at least one bi- or polyfunctional cross-linking agent bound thereto having at least one functional group available for linking the hyaluronic acid to the dextran.

6. The process according to any one of the preceding claims, wherein the polysaccharide product obtained in step (b) is in the form of gel particles having an average size in the range of 0.01-5 mm, preferably 0.1-1 mm.

7. The process according to any one of the preceding claims, wherein said bi- or polyfunctional cross-linking agent is divinyl sulfone or a bis- or polyepoxide.

8. The process according to any one of the preceding claims, wherein said bi- or polyfunctional cross-linking agent is a bis- or polyepoxide.

9. The process according to any one of the preceding claims, wherein said bi- or polyfunctional cross-linking agent is a diglycidyl ether.

10. The process according to any one of the preceding claims, wherein said bi- or polyfunctional cross-linking agent is selected from the group consisting of 1,4-butanediol diglycidyl ether (BDDE), 1,2-bis(2,3-epoxypropoxy)ethylene (EGDGE) and ethylene glycol diglycidyl ether (EGDE), 1,2-ethanediol diglycidyl ether (EDDE) and diepoxyoctane.

11. The process according to any one of the preceding claims, wherein said bi- or polyfunctional cross-linking agent is 1,4-butanediol diglycidyl ether (BDDE).

12. A cross-linked polysaccharide product comprising a hyaluronic acid and a dextran, wherein the hyaluronic acid is in the form of cross-linked gel particles having an average size in the range of 0.01-5 mm, preferably 0.1-1 mm, and the dextran which is a non cross-linked dextran grafted to a surface of cross-linked hyaluronic acid gel particles by ether bonds by means of a bi- or polyfunctional cross-linking agent.

13. A cross-linked polysaccharide product comprising hyaluronic acid and dextran, obtainable by the process according to any one of claims 1-11.

14. A cross-linked polysaccharide product according to any one of claims 12-13 for use as a dermal filler.

## Patentansprüche

1. Verfahren zum Herstellen eines vernetzten Polysacchariderzeugnisses, das Hyaluronsäure und Dextran umfasst, wobei das Verfahren die folgenden Schritte umfasst:
(a) Bereitstellen einer Hyaluronsäure und eines Dextrans;
(b) Binden des Dextrans an die Hyaluronsäure durch Ether- oder Esterbindungen unter Verwendung eines bi- oder polyfunktionellen Vernetzungsmittels;
wobei die in Schritt (a) bereitgestellte Hyaluronsäure ein vernetztes Hyaluronsäuregel ist und das in Schritt (a) bereitgestellte Dextran ein nicht vernetztes Dextran ist;
und wobei Schritt (b) ein Vernetzen des nicht vernetzten Dextrans mit der Hyaluronsäure durch Etherbindungen unter Verwendung eines bi- oder polyfunktionellen Vernetzungsmittels umfasst.

2. Verfahren nach Anspruch 1, wobei die in Schritt (a) bereitgestellte Hyaluronsäure in der Form von Gelpartikeln vorliegt, die eine durchschnittliche Größe in dem Bereich von 0,01-5 mm, vorzugsweise 0,1-1 mm, aufweisen.

3. Verfahren nach einem der vorhergehenden Ansprüche, wobei das in Schritt (a) bereitgestellte Dextran ein Dextran ist, das mit einem bi- oder polyfunktionellen Vernetzungsmittel derart voraktiviert ist, dass das Dextran wenigstens ein daran gebundenes bi- oder polyfunktionelles Vernetzungsmittel umfasst, das wenigstens eine funktionelle Gruppe aufweist, die zum Pfropfen des Dextrans an die Hyaluronsäure verfügbar ist.

4. Verfahren nach einem der vorhergehenden Ansprüche, wobei das in Schritt (a) bereitgestellte Dextran eine durchschnittliche Molekularmasse von weniger als 10 kDa, vorzugsweise weniger als 5 kDa, aufweist.

5. Verfahren nach einem der vorhergehenden Ansprüche, wobei die in Schritt (a) bereitgestellte Hyaluronsäure eine Hyaluronsäure ist, die mit einem bi- oder polyfunktionellen Vernetzungsmittel derart voraktiviert ist, dass die Hyaluronsäure wenigstens ein daran gebundenes bi- oder polyfunktionelles Vernetzungsmittel umfasst, das wenigstens eine funktionelle Gruppe aufweist, die zum Verknüpfen der Hyaluronsäure mit dem Dextran verfügbar ist.

6. Verfahren nach einem der vorhergehenden Ansprüche, wobei das in Schritt (b) erhaltene Polysacchariderzeugnis in der Form von Gelpartikeln vorliegt, die eine durchschnittliche Größe in dem Bereich von 0,01-5 mm, vorzugsweise 0,1-1 mm, aufweisen.

7. Verfahren nach einem der vorhergehenden Ansprüche, wobei das bi- oder polyfunktionelle Vernetzungsmittel Divinylsulfon oder ein Bis- oder Polyepoxid ist.

8. Verfahren nach einem der vorhergehenden Ansprüche, wobei das bi- oder polyfunktionelle Vernetzungsmittel ein Bis- oder Polyepoxid ist.

9. Verfahren nach einem der vorhergehenden Ansprüche, wobei das bi- oder polyfunktionelle Vernetzungsmittel ein Diglycidylether ist.

10. Verfahren nach einem der vorhergehenden Ansprüche, wobei das bi- oder polyfunktionelle Vernetzungsmittel aus der Gruppe ausgewählt ist, die aus 1,4-Butandioldiglycidylether (BDDE), 1,2-Bis(2,3-epoxypropoxy)ethylen (EGDGE) und Ethylenglycoldiglycidylether (EGDE), 1,2-Ethandioldiglycidylether (EDDE) und Diepoxyoctan besteht.

11. Verfahren nach einem der vorhergehenden Ansprüche, wobei das bi- oder polyfunktionelle Vernetzungsmittel 1,4-Butandioldiglycidylether (BDDE) ist.

12. Vernetztes Polysacchariderzeugnis, das eine Hyaluronsäure und ein Dextran umfasst, wobei die Hyaluronsäure in der Form von vernetzten Gelpartikeln vorliegt, die eine durchschnittliche Größe in dem Bereich von 0,01-5 mm, vorzugsweise 0,1-1 mm, aufweisen, und das Dextran ein nicht vernetztes Dextran ist, das an eine Oberfläche von vernetzten Hyaloronsäuegelpartikeln durch Etherbindungen mittels eines bi- oder polyfunktionellen Vernetzungsmittels gepfropft ist.

13. Vernetztes Polysacchariderzeugnis, das Hyaluronsäure und Dextran umfasst, das durch das Verfahren nach einem der Ansprüche 1-11 erhalten werden kann.

14. Vernetztes Polysacchariderzeugnis nach einem der Ansprüche 12-13 zur Verwendung als ein Hautfüller.

## Revendications

1. Procédé de préparation d'un produit polysaccharide réticulé comprenant de l'acide hyaluronique et du dextrane, le procédé comprenant les étapes consistant à :
(a) fournir un acide hyaluronique et un dextrane ;
(b) lier le dextrane à l'acide hyaluronique par des liaisons éther ou ester à l'aide d'un agent de réticulation bi- ou polyfonctionnel ;
dans lequel l'acide hyaluronique fourni à l'étape (a) est un gel d'acide hyaluronique réticulé, et le dextrane fourni à l'étape (a) est un dextrane non réticulé ; et dans lequel l'étape (b) comprend la réticulation du dextrane non réticulé en acide hyaluronique par des liaisons éther à l'aide d'un agent de réticulation bi- ou polyfonctionnel.

2. Procédé selon la revendication 1, dans lequel l'acide hyaluronique fourni à l'étape (a) se présente sous la forme de particules de gel ayant une taille moyenne comprise dans la plage de 0,01 à 5 mm, de préférence de 0,1 à 1 mm.

3. Procédé selon l'une quelconque des revendications précédentes, dans lequel le dextrane prévu à l'étape (a) est un dextrane pré-activé avec un agent de réticulation bi- ou polyfonctionnel de telle sorte que le dextrane comprend au moins un agent de réticulation bi- ou polyfonctionnel lié à celui-ci ayant au moins un groupe fonctionnel disponible pour greffer le dextrane à l'acide hyaluronique.

4. Procédé selon l'une quelconque des revendications précédentes, dans lequel le dextrane fourni à l'étape (a) a un poids moléculaire moyen inférieur à 10 kDa, de préférence inférieur à 5 kDa.

5. Procédé selon l'une quelconque des revendications précédentes, dans lequel l'acide hyaluronique fourni à l'étape (a) est un acide hyaluronique pré-activé avec un agent de réticulation bi- ou polyfonctionnel de telle sorte que l'acide hyaluronique comprend au moins un agent de réticulation bi- ou polyfonctionnel lié à celui-ci ayant au moins un groupe fonctionnel disponible pour la liaison de l'acide hyaluronique au dextrane.

6. Procédé selon l'une quelconque des revendications précédentes, dans lequel le produit polysaccharidique obtenu à l'étape (b) se présente sous la forme de particules de gel ayant une taille moyenne comprise dans la plage de 0,01 à 5 mm, de préférence de 0,1 à 1 mm.

7. Procédé selon l'une quelconque des revendications précédentes, dans lequel ledit agent de réticulation bi- ou polyfonctionnel est la divinyl sulfone ou un bis- ou polyépoxyde.

8. Procédé selon l'une quelconque des revendications précédentes, dans lequel ledit agent de réticulation bi- ou polyfonctionnel est un bis- ou polyépoxyde.

9. Procédé selon l'une quelconque des revendications précédentes, dans lequel ledit agent de réticulation bi- ou polyfonctionnel est un éther de diglycidyle.

10. Procédé selon l'une quelconque des revendications précédentes, dans lequel ledit agent de réticulation bi- ou polyfonctionnel est choisi dans le groupe constitué par le 1,4-butanediol diglycidyl éther (BDDE), le 1,2-bis(2,3-époxypropoxy)éthylène (EGDGE) et l'éther diglycidylique d'éthylène glycol (EGDE), le 1,2-éthanediol diglycidyl éther (EDDE) et le diépoxyoctane.

11. Procédé selon l'une quelconque des revendications précédentes, dans lequel ledit agent de réticulation bi- ou polyfonctionnel est le 1,4-butanediol diglycidyl éther (BDDE).

12. Produit polysaccharidique réticulé comprenant un acide hyaluronique et un dextrane, l'acide hyaluronique se présentant sous la forme de particules de gel réticulé ayant une taille moyenne comprise dans la plage de 0,01 à 5 mm, de préférence de 0,1 à 1 mm, et le dextrane qui est un dextrane non réticulé greffé sur une surface de particules de gel d'acide hyaluronique réticulé par des liaisons éther au moyen d'un agent de réticulation bi- ou polyfonctionnel.

13. Produit de polysaccharide réticulé comprenant de l'acide hyaluronique et du dextrane, pouvant être obtenu par le procédé selon l'une quelconque des revendications 1 à 11.

14. Produit polysaccharidique réticulé selon l'une quelconque des revendications 12 à 13, destiné à être utilisé comme charge dermique.
